# EUROPÄISCHE PATENTSCHRIFT

(11) **EP 1 194 069 B1**
(45) Veröffentlichungstag und Bekanntmachung des Hinweises auf die Patenterteilung: **22.03.2006**
(21) Anmeldenummer: 99936504.2
(22) Anmeldetag: 08.07.1999
(51) Int. Cl.: A61B 5/00

(54) **VORRICHTUNG ZUR MESSUNG DES MENSCHLICHEN BLUTZUCKERSPIEGELS**
DEVICE FOR MEASURING THE BLOOD-SUGAR LEVEL IN HUMANS
DISPOSITIF POUR MESURER LE TAUX DE SUCRE SANGUIN CHEZ L'HOMME

(43) Veröffentlichungstag der Anmeldung: 10.04.2002
(73) Patentinhaber: Leonhardt, Steffen, Dr.-Ing., 23566 Lübeck (DE)
(72) Erfinder: Leonhardt, Steffen, Dr.-Ing., 23566 Lübeck (DE)
(74) Vertreter: Joppich, Martin
(86) Internationale Anmeldenummer: PCT/EP1999/004823
(87) Internationale Veröffentlichungsnummer: WO 2001/003572

(56) Entgegenhaltungen:
- EP-A- 0 554 955
- US-A- 3 837 339
- US-A- 4 900 303
- US-A- 4 975 581
- US-A- 4 979 509
- US-A- 5 048 524

## Beschreibung

Die Erfindung betrifft eine Vorrichtung zur Messung des menschlichen Blutzuckerspiegels nach dem Oberbegriff des Anspruchs 1 sowie eine Vorrichtung zum Regeln des menschlichen Blutzucherspiegels.

Physiologischerweise wird der menschliche Blutzuckerspiegel in gewissen Grenzen konstant gehalten (60 ... 120 mg/dl nüchtern in Ruhe). Der Blutzuckerspiegel wird über hormonelle Regelkreise durch folgende 4 Hormone beeinflußt:
- Insulin (wird in den beta-Zellen der Bauchspeicheldrüse gebildet)
- Glucagon (wird in den Alpha-Zellen der Bauchspeicheldrüse gebildet)
- Adrenalin (wird im Nebennierenmark gebildet)
- Glucocorticoide (z. B. Cortisol, wird in der Nebennierenrinde gebildet)

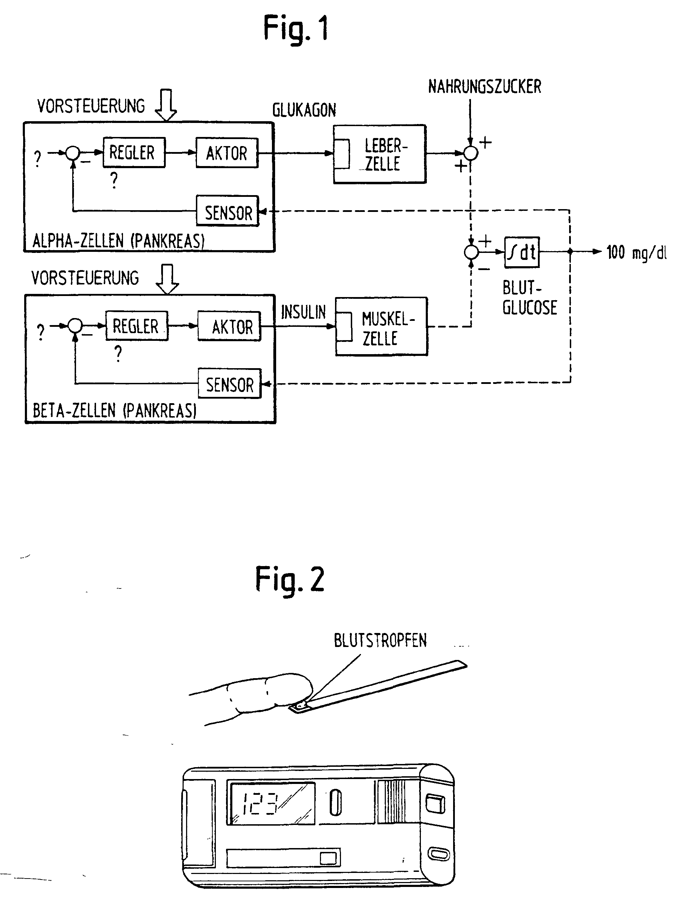

Während Glucagon den Blutzuckerspiegel durch Abbau von Glykogen-Reserven in der Leber erhöht, senkt Insulin den Blutzuckerspiegel durch Transfer des Zuckers aus dem Blut in die verschiedenen Körperzellen. Adrenalin (bei Sport) und Cortisol (bei Streß) wirken langsamer und erhöhen den Blutzuckerspiegel ebenfalls. Demnach ist Insulin das einzige Hormon, das den Blutzuckerspiegel senkt. Fig. 1 zeigt schematisch den Einfluß von Insulin und Glukagon auf den Blutzuckerspiegel.

Die sog. "Zucker"-Krankheit (Diabetes Mellitus) ist durch einen relativen oder absoluten Mangel an Insulin gekennzeichnet, z.B. durch Zerstörung der beta-Zellen im Rahmen einer Autoimmun-Erkrankung (Typ I) oder durch Verlust der Insulin-Wirkung (Insulin-Resistenz, Typ II). Somit ist die Regelung des Blutzuckerspiegels eingeschränkt und als Resultat der Blutzuckerwert im Mittel erhöht. Externe Glucose-Zufuhr (z.B. Nahrungsaufnahme) oder interne Glucose-Bereitstellung (Adrenalin, Cortisol) können beim Diabetiker nicht adäquat ausgeregelt werden, was sich zur Diagnose des Diabetes Mellitus verwenden läßt.

Im Rahmen der heute üblichen Therapie wird der Blutzuckerwert mit Hilfe von Fingerblut ein- oder mehrmals am Tag auf enzymatisch-chemischem Wege mit einem Teststreifen bestimmt. Eine schematische Darstellung eines solchen Testgerätes findet sich in Fig. 2.

Neben diätetischen Maßnahmen (Vergleichsmäßigung der externen Zuckeraufnahme) wird versucht, den Blutzuckerspiegel medikamentös (z.B. durch Sulfonyl-Harnstoff zur Stimulation der vermehrten Insulin-Sekretion) oder durch subkutane Injektion von Insulin in physiologische Bahnen zu lenken. Für die Injektion existieren Injektionshilfen oder mechanische Pumpen zur kontinuierlichen Insulin-Injektion.

Aufgrund der oft nicht ausreichenden Meßhäufigkeit und der fehlenden Möglichkeit, auf Störgrößen adäquat zu reagieren, ist der Blutzuckerspiegel bei einem Großteil der Diabetiker schlecht eingestellt. Bei chronisch zu hohem Blutzuckerspiegel entstehen insbesondere an Nerven und Gefäßen Folgeschäden, die zu drastischen Konsequenzen (Atherosklerose, Herzinfarkt, Amputationen, Erblindung, Dialyse-Pflicht wegen Ausfall der Nierenfunktion, etc.) führen können.

Im experimentellen Stadium befinden sich verschiedene Therapieansätze zur adäquaten Schließung des Blutzucker-Regelkreises bei annehmbarer Regelgüte. Man unterscheidet "biologische" und "technische Konzepte".

Bei den biologischen Konzepten wird versucht, die natürliche Funktion von Spender-Inselzellen als Sensor und Aktor auszunutzen. Klinisch eingesetzt wird die komplette Bauchspeicheldrüsen-Transplantation (in Deutschland ca. 100 p.a.) und die Transplantation von isolierten Spender-Inselzellen (in Deutschland ca. 20 p.a.).

Diese beiden Verfahren sind in ihren Erfolgsaussichten limitiert durch das zu geringe und annähernd konstante Spender-Aufkommen, die notwendige Immun-Suppression mit Infektionsgefahr, die Gefahr der Entstehung bösartiger Tumore und der hohen Medikamentenkosten.

Die Nutzung von unbegrenzt zur Verfügung stehenden Tier-Inselzellen wird durch die noch stärkere Immunreaktion stark behindert.

Bei den technischen Konzepten wird versucht, den defekten Regelkreis durch technische Maßnahmen zu schließen.

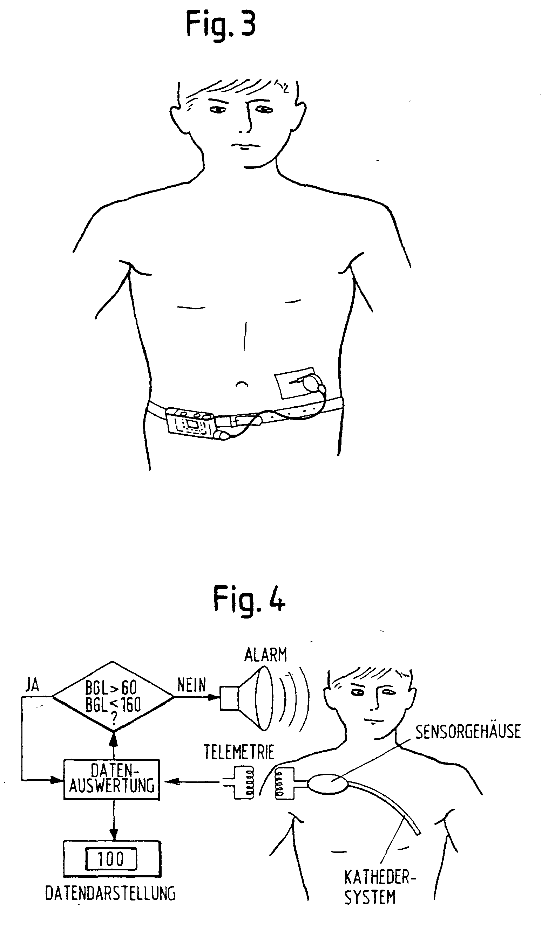

Es ist bekannt, daß als Aktoren Insulin-Pumpen (z.B. Rollen- oder Kolben-Pumpen) verwendet werden können, die sowohl extern als auch als Implantat betrieben werden können. Eine am Patienten befestigte Insulin-Pumpe ist in Fig. 3 dargestellt.

Das zentrale Problem aller technischen Therapieansätze ist die zuverlässige, möglichst kontinuierliche, bequeme und schmerzfreie Bestimmung des Blutzuckerspiegels. Es ist bekannt, daß als Blutzuckersensoren chemische Verfahren eingesetzt werden könne, z.B. das enzymatische Verfahren wie die Glucose-Oxidase (GOD) Reaktion, gegebenenfalls in Kombination mit Ultrafiltration/Mikrodialyse, oder elektrokatalytische Verfahren (direkte Oxidation von Glykose an Platin-Elektroden). Es ist ferner bekannt, daß sich diese Konzepte miniaturisieren und zum Teil auch implantieren lassen. Zum Beispiel existieren implantierbare Prototypen für Mikrodialyse-Sensoren mit GOD-Reation ("Ullmer Zuckeruhr"). Das zentrale Problem aller chemischen Sensor-Prinzipien ist die geringe Standzeit (< 3 Wochen).

Eine gattungsgemäße Vorrichtung ist aus der US 4 975 581 bekannt. Zur Messung des Blutzuckerspiegels wird der Katheter von außen in eine Armvene gelegt, wobei eine Verbindung zu einer Insolinpumpe vorgesehen ist, die nach Auswertung des an dem Blut gestreuten Lichts die daraufhin berechnete Menge Insolin dem Körper zuleitet. Danach muß der Katheter wieder aus der Armvene entfernt werden. Diese Vorrichtung hat den Nachteil, daß durch das notwendige und häufige Einführen des Katheters sich die Einstichstellen entzünden können. Außerdem ist der Patient nach wie vor durch diese Methode sehr in seiner Bewegungsfreiheit behindert. Ein entsprechendes Verfahren, bei dem die Blutzuckerkonzentration durch Auswertung der Polarisation des Lichts bestimmt wird, ist aus der DE 195 40 456 C2 bekannt.

Aus der US 4 704 029 ist eine kombinierte Messung des Blutzuckergehalts durch Auswertung der Absorption, Reflexion und Polarisation von Licht an dem Blut bekannt. Grundsätzlich ist diese Vorrichtung implantierbar. Ein wesentliches Problem ist allerdings die Tatsache, das die Meßfläche durch Ablagerungen mit der Zeit verschmutzt, so daß die Meßwerte unbrauchbar werden.

Aus der DE 37 36 092 A1 ist eine Meßvorrichtung zur kontinuierlichen Bestimmung der Konzentration des Blutzuckergehalts in einer Meßküvette bekannt, bei der durch Ermittlung der optischen Rotation durch Polarisator und Analysator eine Ermittlung der Blutzuckerkonzentration erfolgt. Eine entsprechende Vorrichtung mit Auswertung der Reflexion des Lichts an dem Blut ist aus der WO 97/28437 bekannt.

Aus der WO 91/18548 ist eine Vorrichtung bekannt, bei der von außen durch die Haut eine Bestimmung des Blutzuckerspiegels durchgeführt wird, wobei zwei Infrarot-Wellenlängen ausgesendet und empfangen werden und die Absorption der Wellenlängen ausgewertet wird. Die Genauigkeit dieser nichtinvasiven "in vivo" Messung reicht allerdings wegen der Absorption durch Haut- bzw. Schleimhautzellen für den klinischen Einsatz nicht aus. Ein entsprechendes Verfahren durch Auswertung eines Teststrahls und eines Referenzstrahls ist aus der US 5 146 091 bekannt. US 5 048 524 A offenbart eine Vorrichtung mit den Merkmalen des Oberbegriffs des Anspruchs 1.

Aufgabe der Erfindung ist es, eine Vorrichtung der gattungsgemäßen Art bereitzustellen, die möglichst genaue und über die Zeit beständige Meßwerte des Blutzuckergehalts liefert, die zur weiteren Datenverarbeitung zugrundegelegt werden können.

Die Erfindung ist in den Ansprüchen definiert.

Die Vorrichtung weist eine an der Meßstelle vorgesehene Reinigungsvorrichtung zur Entfernung von aus dem Blut abgelagerten Gewebeteilen auf. Die aufgrund der Immunabwehr des Blutes ablaufenden Prozesse führen zu einer wesentlichen Beeinträchtigung der Messung. Aufgrund der Immunabwehr werden beispielsweise nicht zerstörbare Fremdkörper durch körpereigenes Gewebe eingekapselt. Die Vorrichtung ermöglicht es, dieses körpereigene Gewebe vor der Messung zu entfernen, wobei die Reinigung an der Meßstelle kontinuierlich oder diskontinuierlich erfolgen kann. Zur diskontinuierlichen Reinigung ist die Reinigungsvorrichtung zweckmäßigerweise seitens der Recheneinheit ansteuerbar.

Nach einer Ausführungsform, die nicht einen Teil der vorliegenden Erfindung bildet, ist vorgesehen, daß die Reinigungsvorrichtung aus einer Spülvorrichtung besteht, mit der die Meßstelle durch eine Spülflüssigkeit freigespült wird. Die Spülflüssigkeit kann beispielsweise aus einer physiologischen Kochsalzlösung bestehen. Hierzu befinden sich Auslaßöffnungen der Flüssigkeit direkt vor dem Meßfenster. Nach der Spülung sollte eine ausreichende Zeit bis zur nächsten Messung abgewartet werden.

Erfindungsgemäß ist vorgesehen, daß die Reinigungsvorrichtung aus einem ansteuerbaren Aktor besteht, der eine Lichtaustrittsöffnung des mindestens einen Lichtleiters bei Ansteuerung freigibt oder freiwischt. Beispielsweise kann die Reinigung durch eine formschlüssige Relativbewegung eines Körpers gegen den Meßort erfolgen. Beispielsweise kann der Aktor aus einem Kolben bestehen, der in einer passgenauen Öffnung am freien Ende des Katheders zwischen einer bündig mit der Katheter-Oberfläche abschließenden Stellung und einer gegenüber der Katheder-Oberfläche versenkten Stellung geführt ist, und der in der versenkten Stellung eine Lichtaustrittsöffnung des mindestens einen Lichtleiters freigibt. Der Kolben kann dabei radial oder axial in dem Katheter geführt sein.

Als Antriebsprinzipien für den Aktor kommt eine Form-Gedächtnis-Legierung, ein thermopneumatischer Antrieb, ein elektrostatischer Antrieb durch ein Piezo-Element oder auch ein Rotor in Betracht. Soweit der Aktor ansteuerbar ist, kann die Steuerleitung aus einer hydraulischen oder pneumatischen Leitung bestehen, die mit dem Kolben in Wirkverbindung steht, wobei in dem implantierbaren Gehäuse ein Hubantrieb zur Beaufschlagung eines Druckes auf die Steuerleitung integriert ist. Der Kolben kann beispielsweise durch einen Mikromotor mit einem Kurbelantrieb antreibbar sein, wobei die Steuerleitung dann aus einer elektrischen Steuerleitung bestehen kann. Ebenfalls ist es denkbar, daß der Kolben durch einen Hubmagneten antreibbar ist.

Nach einer weiteren Ausführungsform der Erfindung ist vorgesehen, daß die Reinigungsvorrichtung ein ansteuerbarer Ultraschallgenerator ist, der Ultraschallwellen oder Stoßwellen bei Ansteuerung derart aussendet, daß die Lichtaustrittsöffnung des mindestens einen Lichtleiters bei Ansteuerung von Ablagerungen befreit wird.

Nach einer weiteren Ausführungsform, die nicht einen Teil der vorliegenden Erfindung bildet, ist vorgesehen, daß die Reinigungsvorrichtung eine elektrostatische Einheit zur Erzeugung von elektrostatischen Kräften umfaßt, mit der eine definierte Ladung an den Meßort gebracht wird.

Nach einer weiteren Ausführungsform, die nicht einen Teil der vorliegenden Erfindung bildet, kann vorgesehen sein, daß die Reinigungsvorrichtung eine Einheit zur Erzeugung hochenergetischer Lichtwellen umfaßt, die eine gezielte lokale Verdampfung von abgelagerten Gewebeteilen bewirkt. Zweckmäßigerweise kann für die Einstrahlung der hochenergetischen Lichtwellen der Lichtleiter selbst verwendet werden. Möglich sind allerdings auch zusätzliche Lichtleiter im Katheter-Inneren.

Die zur Ansteuerung nötige Energie kann in elektrischer, thermischer, optischer, mechanischer, hydraulischer oder pneumatischer Form an die Meßstelle gebracht werden.

Die Messung an der Meßstelle kann durch Transmission, also aufgrund Durchstrahlung einer definierten Meßstrecke, oder durch diffuse Reflektion von der Katheter-Oberfläche erfolgen. Bei Transmission wird es in der Regel erforderlich sein, einen hinführenden und einen rückführenden Lichtleiter vorzusehen, wobei der Lichtweg zwischen dem hinführenden und dem rückführenden Lichtleiter an der Meßstelle zu Meßzwecken unterbrochen ist. Bei Messung in Transmission erfolgt die Reinigung der Meßstelle typischerweise zwischen zwei Lichtleitersegmenten. Bei Messung in Reflektion liegt die Reinigungsvorrichtung vor der Lichtaus- und eintrittsstelle (Meßoptik).

Zur medizinischen Verträglichkeit ist der Katheter mit einem biokompatiblen Material ummantelt. Im Katheter befindet sich dabei mindestens ein Lichtleiter, wobei der Katheter und der Lichtleiter auch einstückig aus einem Vollmaterial ausgeführt sein können. Am Meßort ist die Ummantelung unterbrochen oder gefenstert.

Nach einer weiteren bevorzugten Ausführungsform ist vorgesehen, daß die Lichtquelle, der Detektor und die Recheneinheit zusammen mit einem Energiespeicher zur Spannungsversorgung in einem Implantat integriert sind. Die Meßstelle im Blut ist damit von der eigentlichen optischen Meßvorrichtung räumlich getrennt und durch Lichtleiter im Inneren des Katheters optisch verbunden.

Nach einer weiteren bevorzugten Ausführungsform ist vorgesehen, daß in dem Implantat eine Telemetrie-Einheit integriert ist, mit der zwischen der Recheneinheit und einer außerhalb des Körpers befindlichen Kontrolleinheit mit einem dafür vorgesehenen Tastkopf eine Datenübertragung und/oder eine Energieübertragung herstellbar ist. Die Telemetrie-Einheit und der Tastkopf können beispielsweise einen Schwingkreis mit jeweils einer Induktivität aufweisen, wobei der Schwingkreis seitens der Telemetrie-Einheit und der Schwingkreis seitens des Tastkopfes zur Datenübertragung verstimmbar sind. Die Datenübertragung kann auf diese Weise berührungslos durch induktive Kopplung der Induktivitäten erfolgen. Besonders zweckmäßig ist es, daß der Energiegehalt, der von der Kontrolleinheit zur Telemetrie-Einheit übertragenen Frequenz einen Akkumulator oder einen Kondensator zur Energieversorgung des Implantats auflädt.

Eine Vorrichtung zum Regeln des menschlichen Blutzuckerspiegels, für die ebenfalls selbstständiger Schutz beansprucht wird, weist die Merkmale des Anspruchs 20 auf. Die erfindungsgemäßen Vorrichtungen lassen sich implantieren, so daß eine Regelung des menschlichen Blutzuckerspiegels durchgeführt werden kann, wobei die Meßwerte über eine Telemetrieeinheit zu einer außerhalb des Körpers befindlichen Kontrolleinheit übertragen werden, mit einer extrakorporalen Insulinpumpe zum Injektieren von Insulin über das Bauchfell und mit einem in der Kontrolleinheit integrierten Regler, der in Abhängigkeit der Meßwerte die Insulinpumpe derart steuert, daß sich der gewünschte Blutzuckerspiegel einstellt.

Eine weitere Lösung der oben genannten Aufgabe läßt sich für eine gattungsgemäße Vorrichtung zur Messung des menschlichen Blutzuckerspiegels dadurch erreichen, daß ein Ultraschallgenerator vorgesehen ist, mit dem Ultraschallwellen derart aussendbar sind, daß die Meßstelle von aus dem Blut abgelagerten Gewebeteilen befreit wird. Selbst wenn die gattungsgemäße Vorrichtung damit als Implantat ausgeführt ist, kann auf diese Weise eine Reinigung der Meßstelle durch einen Ultraschallgenerator außerhalb des Körpers erfolgen. Beispielsweise ist es denkbar, daß die Recheneinheit bei jeder Auswertung das Meßergebnis nach bestimmten Charakteristiken auswertet, aus denen sich bestimmen läßt, ob eine Störung des Meßvorgangs aufgrund einer Verschmutzung der Meßstelle vorliegt. Sobald das Meßergebnis aufgrund der festgestellten Charakteristiken in unzulässiger Weise verfälscht ist, wird ein Alarmsignal ausgegeben, wodurch der Patient veranlaßt wird, eine Reinigung der Meßstelle durch die externe Vorrichtung vornehmen zu lassen.

Weitere Einzelheiten der Erfindung werden anhand der Zeichnung erläutert. In dieser zeigt:
- Fig. 1: schematisch den Einfluß von Insulin und Glukagon auf den Blutzuckerspiegel,
- Fig. 2: eine schematische Darstellung eines bekannten Testgerätes,
- Fig. 3: eine am Patienten befestigte Insulin-Pumpe,
- Fig. 4: das erfindungsgemäße Meßsystem zur kontinuierlichen Bestimmung des Blutzuckerspiegels,
- Fig. 5: das Implantat mit Katheter, der einen integrierten Lichtleiter und an seinem freien Ende einen Mikrokolben aufweist und
- Fig. 6: den Einsatz des erfindungsgemäßen Implantats im geschlossenen Regelkreis,
- Fig. 7: zeigt ein elektrisches Prinzipschaltbild für die Datenübertragung von der in dem Implantat befindlichen Telemetrieeinheit zu der Kontrolleinheit,
- Fig. 8: zeigt ein elektrisches Prinzipschaltbild für die Datenübertragung von der Kontrolleinheit zu der Telemetrieeinheit in dem Implantat,
- Fig. 9: zeigt eine vergrößerte Darstellung des mikromechanischen Kurbelantriebs gemäß Fig. 5,
- Fig. 10: zeigt die erfindungsgemäße Vorrichtung mit einem Ultraschallgenerator und
- Fig. 11: zeigt eine Vorrichtung mit zwei Lichtleitern und einer Messung basierend auf diffuser Reflektion.

Die Figuren 1, 2 und 3 wurden bereits oben beschrieben.

Fig. 4 zeigt das erfindungsgemäße Blutzuckermeßsystem.

Das Meßsystem besteht aus einem Sensor-Gehäuse aus bioverträglichem Material (z.B. Titan) und ein mit Körperflüssigkeiten (z.B. Blut) in Kontakt stehendes Kathetersystem aus bioverträglichem Material (z.B. Silikon). Zum Meßsystem gehören ferner eine extrakorporale Sende-/Empfangseinheit mit Telemetrie, Datenauswertung, Datendarstellung und Alarmfunktion.

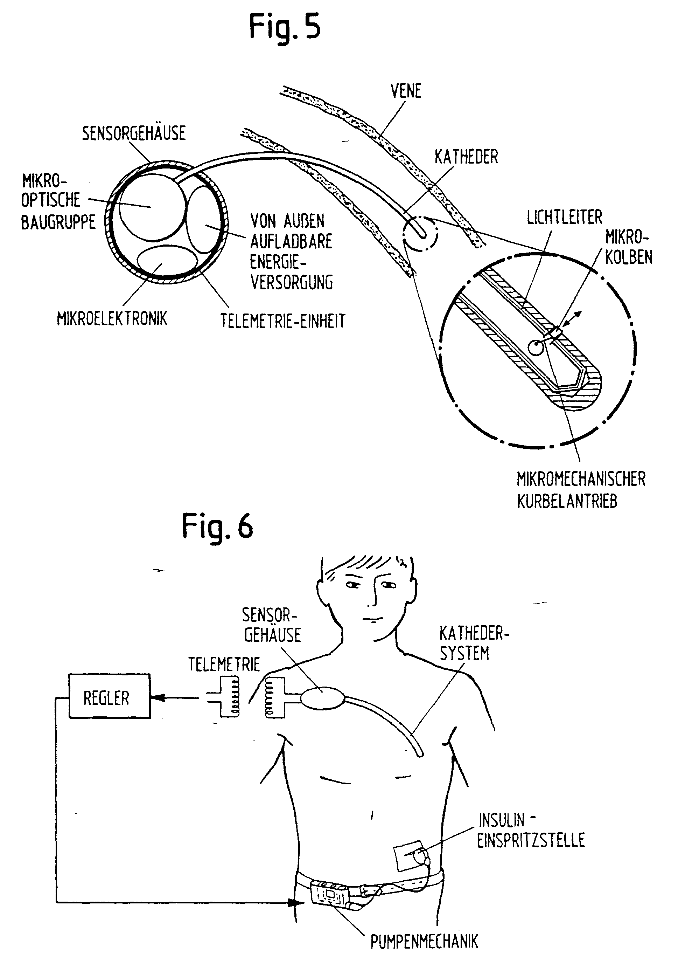

Fig. 5 zeigt das Implantat mit Katheter, der einen integrierten Lichtleiter und an seinem freien Ende einen Mikrokolben aufweist.

Im Sensor-Gehäuse sind mehrere Funktionsgruppen integriert:
- transkutane Telemetrie-Einheit zur bidirektionalen Optischen oder elektromagnetischen Datenübertragung zwischen implantiertem Sensor und extrakorporaler Sende-/Empfangseinheit.
- (von außen aufladbare) Energieversorgung
- integrierte Mikroelektronik/Mikrorechner zur Steuerung aller Vorgänge im Implantat (Messungen, Signalauswertung, Telemetrie, Selbstdiagnose, Überwachung der Batteriefunktion, etc.)
- integrierte mikrooptische Baugruppe zur Erzeugung und Messung von Absorption, Reflexion und Polarimetrie elektromagnetischer Wellen in verschiedenen Wellenbereichen (z.B. sichtbares Licht oder nahes/mittleres Infrarot-Licht). Innerhalb eines Wellenbereiches können verschiedene Frequenzen realisiert werden, z.B. durch mehrere Lichtquellen, durch mikrooptische Prismen oder durch durchstimmbare optische Filter.

Der Katheter ist mehrlumig und enthält einen oder mehrere Lichtleiter, die das Licht von und zu der mikrooptischen Baugruppe im Sensorgehäuse leiten. Ferner kann der Katheter elektrische Leitungen zur Energieübertragung, Bauteile zur mechanischen Kraftübertragung (z.B. Bowdenzüge) oder Lumen zur hydraulischen oder pneumatischen Energieübertragung enthalten. Der Katheter steht an den Meßstellen in Kontakt mit Körperflüssigkeiten (z.B. Blut, Peritonealflüssigkeit oder Liquor).

Bei der Absorptions- und der Polarimetrie-Messung wird Licht verschiedener Frequenzen nacheinander längs einer konstanten Wegstrecke durch die Meßprobe geleitet. Hierzu wird das Probenvolumen durch einen dicht abschließenden, beweglichen Mikrokolben vor der Messung in den Katheter gesaugt und nach der Messung ausgestoßen. Zur Erzeugung der Kolbenbewegung kann ein mikromechanischer Kurbeltrieb eingesetzt werden

Aber auch mechanische, hydraulische, pneumatische oder thermopneumatische Prinzipien zur Bewegungserzeugung sind denkbar. Durch die Kolbenbewegung werden die Lichtein- und Austrittstellen automatisch gesäubert und eine Gerinnung von Blut oder Ablagerung von Eiweiß in der Meßstrecke verhindert. Ferner sorgen die Kolbenbewegungen bei hinreichend geringem zeitlichen Abstand (z.B. alle 10 min.) dafür, daß sich direkt über der Meßstrecke bei Messung im Blut keine fibrinöse Pseudomembran bilden kann.

Bei der Reflektionsspektroskopie muß die Wegstrecke durch die Meßprobe nicht konstant sein. Vielmehr wird das Licht durch eine geeignete Mikrooptik aus dem Katheter in die Flüssigkeit geleitet und diffus gestreut. Über eine zweite Mikrooptik wird ein Teil des Streulichtes eingefangen und zurück an das Sensor-Gehäuse geleitet. Um die Bildung von Ablagerungen und Pseudomembranen über den Mikrooptiken zu verhindern, besteht wiederum die Möglichkeit, Licht-Ein-und -austrittsstellen über einen beweglichen Kolben zu reinigen. Eine Reinigung der Mikrooptiken durch Ultraschall-Transducer ist ebenfalls möglich. Bei entsprechender Plazierung des Katheters (rechtes Atrium des Herzens, wie bei Herzschrittmachern) läßt sich auch die Bewegung der Herzklappensegel (Tricuspedal-Klappe) als "Scheibenwischer" nutzen.

Zur Erhöhung der Meßgenauigkeit und -sicherheit lassen sich alle drei Meßverfahren (Absorption, Reflektion und Polarimetrie) in geeigneter Weise kombinieren und entsprechend gewichten.

Das Sensor-Gehäuse ist hinreichend klein und kann typischerweise in einer Gewebetasche unterhalb des Schlüsselbeins (subclavikulär, typ. Implantationsort für Herzschrittmacher) oder im umbilikalen Unterhautfettgewebe implantiert werden.

Fig. 6 zeigt den Einsatz des erfindungsgemäßen Implantats im geschlossenen Regelkreis. Das mikrooptische BlutzuckerMeßsystem (implantierter Katheter und implantiertes Sensor-Gehäuse sowie externe Sende-/Empfangseinheit) kann neben der Überwachungsfunktion natürlich auch zur Schließung des defekten Blutzuckerregelkreises verwendet werden. Typischerweise wird dabei das Meßsystem mit einem Regelalgorithmus und entsprechender extrakorporaler Hardware (Elektronik, Pumpenmechanik) kombiniert.

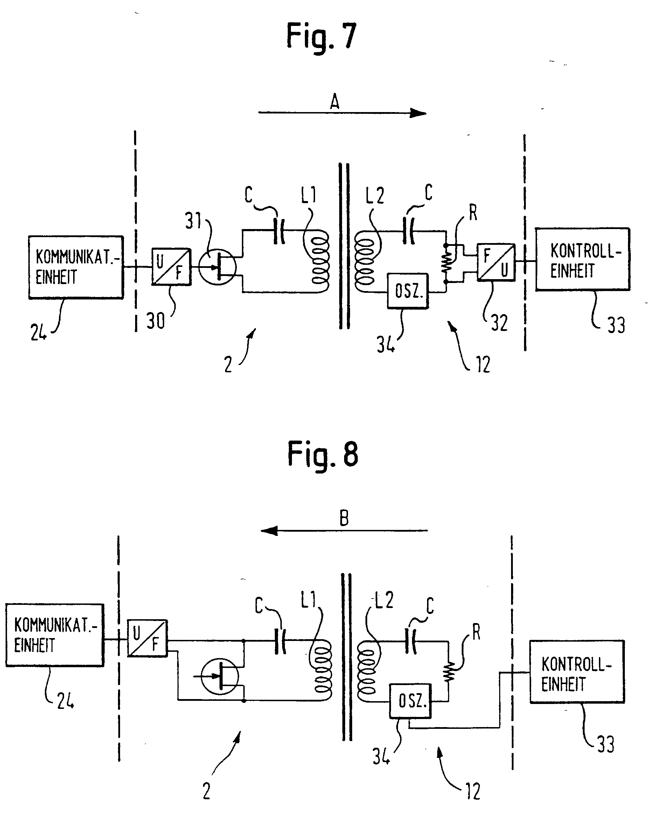

Fig. 7 zeigt ein elektrisches Prinzipschaltbild für die Datenübertragung von der in dem Implantat befindlichen Telemetrieeinheit zu der Kontrolleinheit. Die Übertragungsrichtung ist durch den Pfeil A angedeutet. Die in der Kommunikationseinheit 24 gewandelten Werte werden dem Tastkopf 2 und dort einem Spannungs-Frequenzwandler 30 zugeführt, der einen Schalttransistor 31 taktet, wodurch die Resonanzfrequenz des durch C und L1 gebildeten Schwingkreises entsprechend der Taktfrequenz verändert wird. In dem Tastkopf 12 wird hierdurch die Resonanzfrequenz des durch C und L2 gebildeten Schwingkreises verstimmt, wobei die Sendeenergie durch den in dem Tastkopf 12 befindlichen Oszillator 34 geliefert wird. Über den Widerstand R wird die derart modulierte Resonanzfrequenz über den Frequenz-Spannungswandler in Form einer analogen Spannung der Kontrolleinheit 33 zugeführt.

Fig. 8 zeigt ein elektrisches Prinzipschaltbild für die Datenübertragung von der Kontrolleinheit zu der Telemetrieeinheit in dem Implantat. Die Übertragungsrichtung ist durch den Pfeil B angedeutet. In diesem Fall wird die Frequenz des Oszillators 34 entsprechend den zu übertragenden Werten der Kontrolleinheit 33 verändert. Die Datenübertragung erfolgt ansonsten analog gemäß Fig. 3.

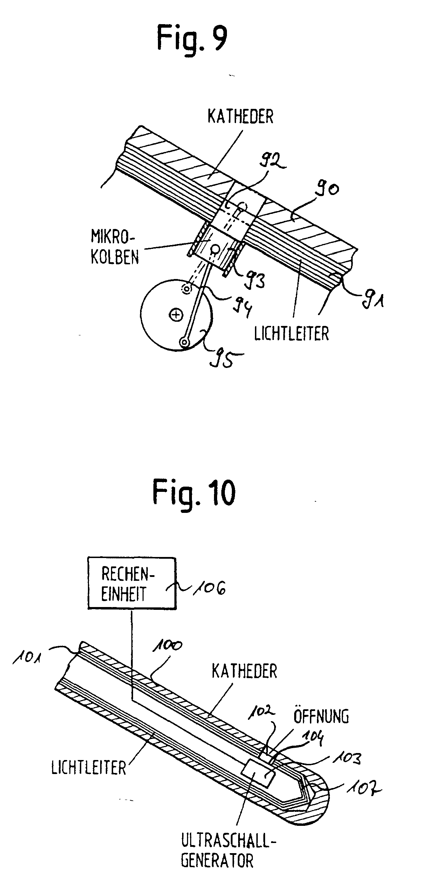

Fig. 9 zeigt eine vergrößerte Darstellung des mikromechanischen Kurbelantriebs gemäß Fig. 5. Der Lichtleiter 91 ist direkt mit einem biokompatiblen Material 90 ummantelt, wobei die Messung in Transmission über die Meßstelle 92 erfolgt. Die Meßstelle 92 kann durch formschlüssige Bewegung des Kolbens 93 gereinigt werden, wobei der Kolben 93 über eine Kurbelstange 94 durch eine Kurbelscheibe 95 angetrieben wird. Der Antrieb der Kurbelscheibe 95 kann entweder direkt durch einen Mikromotor erfolgen oder aber extrakorporal durch eine Kraftübertragung auf die Kurbelscheibe. Bei einer extrakorporalen Kraftübertragung ist es beispielsweise denkbar, daß die Kurbelscheibe 95 mit entsprechenden Magneten besetzt ist, so daß die Kurbelscheibe einem außen angelegten, rotatorischen Magnetfeld in einer entsprechenden Drehung folgt.

Fig. 10 zeigt die erfindungsgemäße Vorrichtung mit einem Ultraschallgenerator (103) vor der Meßstelle. Der Katheter 100 besteht wiederum aus einem biokompatiblen Material, an dessen Innenwandung der Lichtleiter 101 angebracht ist. An der Meßstelle 102 ist der Lichtleiter unterbrochen, so daß eine Transmissionsmessung der dort befindlichen Blutmenge vorgenommen werden kann. Am Ende des Katheters ist der Lichtleiter an der Stelle 107 entsprechend abgeknickt, um eine bessere Umleitung des Lichtstrahls zu ermöglichen. Vor der Meßstelle im Inneren des Katheters findet sich ein Ultraschallgenerator 103, der durch eine Recheneinheit 106 angesteuert wird. An der Meßstelle 102 kann im Inneren des Katheters eine Membran 104 vorgesehen sein, die durch Anregung des Ultraschallgenerators in Schwingungen versetzt wird, wodurch die Meßstelle 102 von aus dem Blut abgelagerten Gewebeteilen gereinigt wird. Es ist aber auch denkbar, daß der Ultraschallgenerator direkt an der Innenwandung des Lichtleiters 101 angesetzt ist, so daß Stoßwellen direkt in die Meßstelle 102 ausgesendet werden.

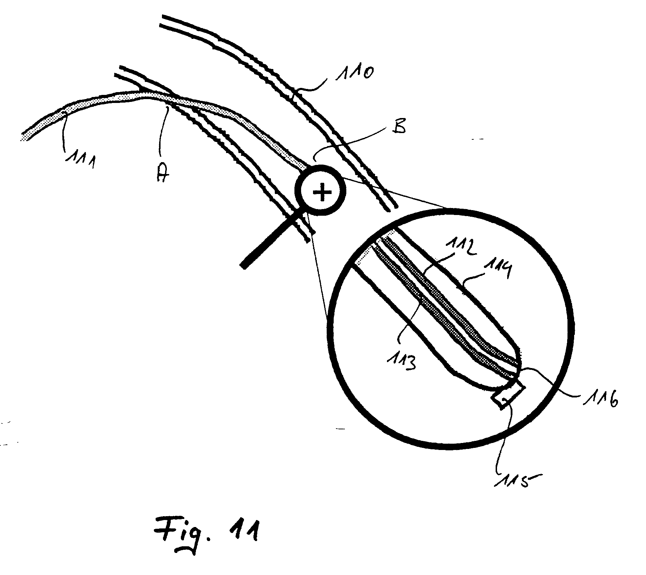

Fig. 11 zeigt eine Vorrichtung basierend auf diffuser Reflektion. In ein Blutgefäß 110 ist der Katheter 111 an der Stelle A eingeführt. Das freie Ende B des Katheters ist vergrößert dargestellt. Der Katheter besteht demnach aus einer Ummantelung 114 aus biokompatiblem Material sowie zwei im Inneren geführten Lichtleitern 112 und 113, die beide parallel an die Meßstelle 116 geführt sind. Am Ende der Meßstelle 116 ist eine Reinigungsvorrichtung 115 vorgesehen, die wie oben beschrieben ausgeführt sein kann.

## Patentansprüche

1. Vorrichtung zur Messung des menschlichen Blutzuckerspiegels,
mit einem Katheter, dessen freies Ende in ein Blutgefäß gelegt werden kann, obei der Katheter mindestens einen Lichtleiter umfasst,
mit einer Lichtquelle zum Einkoppeln von Licht in den mindestens einen Lichtleiter,
mit einer Messstelle am freien Ende des Katheters, an der das Licht aus dem mindestens einen Lichtleiter austritt, wobei das Licht vom Blut gestreut und/oder durch das Blut geleitet wird und wobei das gestreute und/oder durchgeleitete Licht in den mindestens einen Lichtleiter wieder eingekoppelt wird,
mit einem Detektor zur Aufnahme des zurückgeführten Lichtes,
mit einer Recheneinheit zur Auswertung des von dem Detektor aufgenommenen Lichts, und
mit einer an der Messstelle vorgesehenen Reinigungsvorrichtung zur Entfernung von aus dem Blut abgelagerten Gewebeteilen,
**dadurch gekennzeichnet,**
**dass** die Reinigungsvorrichtung aus einem ansteuerbaren Aktor besteht, der eine Lichtaustrittsöffnung des mindestens einen Lichtleiters bei Ansteuerung freigibt oder freiwischt.

2. Vorrichtung gemäß Anspruch 1, **dadurch gekennzeichnet, dass** die Reinigungsvorrichtung seitens der Recheneinheit ansteuerbar ist.

3. Vorrichtung nach einem der Ansprüche 1 - 2, **dadurch gekennzeichnet, dass** die Bewegung des Aktors durch eine Form-Gedächtnis-Legierung, durch einen thermopneumatischen Antrieb, durch einen elektrostatischen Antrieb oder durch einen Rotor erfolgt.

4. Vorrichtung nach einem der Ansprüche 1 - 3, **dadurch gekennzeichnet, dass** der Aktor aus einem Kolben besteht, der in einer passgenauen öffnung am freien Ende des Katheters zwischen einer bündig mit der Katheter-Oberfläche abschließenden Stellung und einer gegenüber der Katheter-Oberfläche versenkten Stellung geführt ist und daß in der versenkten Stellung eine Lichtaustrittsöffnung des mindestens einen Lichtleiters freigegeben ist.

5. Vorrichtung nach Anspruch 4, **dadurch gekennzeichnet, dass** der Kolben radial in dem Katheter geführt ist.

6. Vorrichtung nach Anspruch 4, **dadurch gekennzeichnet, dass** der Kolben axial in dem Katheter geführt ist.

7. Vorrichtung nach einem der Ansprüche 4 - 6, **dadurch gekennzeichnet, dass** der Kolben durch einen Mikromotor mit Kurbelantrieb antreibbar ist und dass die Steuerleitung aus einer elektrischen Steuerleitung besteht.

8. Vorrichtung nach einem der Ansprüche 4 - 6, dass der Kolben durch einen Hubmagneten antreibbar ist.

9. Vorrichtung nach einem der Ansprüche 1 - 8, **dadurch gekennzeichnet, dass** die Ansteuerung durch eine elektrische Steuerleitung erfolgt.

10. Vorrichtung nach einem der Ansprüche 1 - 8, **dadurch gekennzeichnet, dass** die Steuerleitung aus einer hydraulischen oder pneumatischen Leitung besteht, die mit dem Kolben in Wirkverbindung steht, wobei in dem implantierbaren Gehäuse ein Hubantrieb zur Beaufschlagung eines Druckes auf die Steuerleitung integriert ist.

11. Vorrichtung nach dem Oberbegriff des Patentanspruchs 1, **dadurch gekennzeichnet, dass** die Reinigungsvorrichtung ein ansteuerbarer Ultraschallgenerator ist, der Ultraschallwellen bei Ansteuerung derart aussendet, dass die Lichtaustrittsöffnung des mindestens einen Lichtleiters bei Ansteuerung von Ablagerungen befreit wird.

12. Vorrichtung nach einem der Ansprüche 1 - 11, **dadurch gekennzeichnet, dass** die zur Ansteuerung nötige Energie in elektrischer, thermischer, optischer, mechanischer, hydraulischer oder pneumatischer Form an die Messstelle gebracht wird.

13. Vorrichtung nach einem der Ansprüche 1 - 12, **dadurch gekennzeichnet, dass** die Messung an der Messstelle durch Transmission oder durch diffuse Reflexion von der Katheteroberfläche erfolgt.

14. Vorrichtung nach einem der Ansprüche 1 - 13, **dadurch gekennzeichnet, dass** der Katheter mit einem biokompatiblen Material ummantelt ist.

15. Vorrichtung nach Anspruch 14, **dadurch gekennzeichnet, dass** Katheter und Lichtleiter einstückig aus einem Vollmaterial ausgeführt sind.

16. Vorrichtung nach einem der Ansprüche 1 - 15, **dadurch gekennzeichnet, dass** die Lichtquelle, der Detektor und die Recheneinheit zusammen mit einem Energiespeicher zur Spannungsversorgung in einem Implantat integriert sind.

17. Vorrichtung nach Anspruch 16, **dadurch gekennzeichnet, dass** in dem Implantat eine Telemetrie-Einheit integriert ist, mit der zwischen der Recheneinheit und einer außerhalb des Körpers befindlichen Kontrolleinheit mit einem dafür vorgesehenen Tastkopf eine Datenübertragung und/oder eine Energieübertragung herstellbar ist.

18. Vorrichtung nach Anspruch 17, **dadurch gekennzeichnet, dass** die Telemetrie-Einheit und der Tastkopf einen Schwingkreis mit jeweils einer Induktivität aufweisen, wobei der Schwingkreis seitens der Telemetrie-Einheit und der Schwingkreis seitens des Tastkopfes zur Datenübertragung verstimmbar sind und dass die Datenübertragung berührungslos durch induktive Kopplung der Induktivitäten erfolgt.

19. Vorrichtung nach Anspruch 17, **dadurch gekennzeichnet, dass** der Energiegehalt der von der Kontrolleinheit zur Telemetrie-Einheit übertragenen Frequenz einen Akkumulator oder einen Kondensator zur Energieversorgung des Implantats auflädt.

20. Vorrichtung zum Regeln des menschlichen Blutzuckerspiegels,
mit einer implantierbaren Vorrichtung nach einem der Ansprüche 16 bis 19 zum Messen des menschlichen Blutzuckerspiegels, wobei die Messwerte über eine Telemetrie-Einheit zu einer außerhalb des Körpers befindlichen Kontrolleinheit übertragen werden können,
mit einer extrakorporalen Insulinpumpe zum Injektieren von Insulin über das Bauchfell und
mit einem in der Kontrolleinheit integrierten Regler, der in Abhängigkeit der Messwerte die Insulinpumpe derart steuert, dass sich der gewünschte Blutzuckerspiegel einstellt.

## Claims

1. Apparatus for measuring human blood sugar levels, comprising:
a catheter, the free end of which can be positioned in a blood vessel, wherein the catheter comprises at least one optical waveguide,
a light source for coupling light into the at least one optical waveguide,
a measuring point at the free end of the catheter at which point the light is emitted from the at least one optical waveguide, wherein the light is dispersed by the blood and/or transmitted through the blood, and wherein the dispersed and/or transmitted light is coupled again into the at least one optical waveguide,
a detector to receive the light which is returned,
a computer unit for analysing the light received by the detector, and
a cleansing device located at the measuring point for removing the tissue particles deposited from the blood,
**characterized in that**,
the cleansing device consists of a controllable actuator which frees or wipes clear a light emission orifice of the at least one optical waveguide when activated.

2. Apparatus according to claim 1, **characterized in that** the cleansing device is controllable by the computer unit.

3. Apparatus according to one of the claims 1 - 2, **characterized in that** the movement of the actuator is generated by a shape memory alloy, a thermopneumatic drive, an electrostatic drive or a rotor.

4. Apparatus according to one of the claims 1 - 3, **characterized in that** the actuator consists of a piston which is inserted into a form-fitting opening located at the free end of the catheter and which moves between a position where it forms a seal flush with the catheter surface and a position where it fits into a recess located opposite the catheter surface, and that in the recessed position a light emission orifice of the at least one optical waveguide becomes free.

5. Apparatus according to claim 4, **characterized in that** the piston is guided radially within the catheter.

6. Apparatus according to claim 4, **characterized in that** the piston is guided axially within the catheter.

7. Apparatus according to one of the claims 4 - 6, **characterized in that** the piston can be actuated by a micromotor with a crank drive and that the control line consists of an electrical control line.

8. Apparatus according to one of the claims 4 - 6, **characterized in that** the piston can be actuated by a lifting magnet.

9. Apparatus according to one of the claims 1 - 8, **characterized in that** the control action is achieved by means of an electrical control line.

10. Apparatus according to one of the claims 1 - 8, **characterized in that** the control line consists of a hydraulic or pneumatic line which is effectively connected to the piston, whereby a linear actuator is integrated in the implantable housing to exert a force on the control line.

11. Apparatus according to the preamble of claim 1, **characterized in that** the cleansing device is constituted by a controllable ultrasonic generator which emits ultrasonic waves when activated in such a way that the light emission orifice of the at least one optical waveguide is freed of deposition when activation occurs.

12. Apparatus according to one of the claims 1 - 11, **characterized in that** the energy required for control purposes is supplied to the measuring point in electrical, thermal, optical, mechanical, hydraulic or pneumatic form.

13. Apparatus according to one of the claims 1 - 12, **characterized in that** the measurement at the measuring point is undertaken by means of transmission or by means of diffuse reflection from the surface of the catheter.

14. Apparatus according to one of the claims 1 - 13, **characterized in that** the catheter is clad with a biocompatible material.

15. Apparatus according to claim 14, **characterized in that** the catheter and the optical waveguide are designed as a single piece of solid material.

16. Apparatus according to one of the claims 1 - 15, **characterized in that** the light source, the detector and the computer unit are integrated into the implant together with an energy store for providing voltage.

17. Apparatus according to claim 16, **characterized in that** a telemetry unit is integrated into the implant with which it is possible to transmit data and/or energy between the computer unit and a control unit located extracorporeally with a probe for that purpose.

18. Apparatus according to claim 17, **characterized in that** the telemetry unit and the probe have an oscillatory circuit each with an inductor, wherein the oscillatory circuit on the side of the telemetry unit and the oscillatory circuit on the side of the probe can be tuned for the transmission of data and **characterized in that** the data are transmitted without direct contact by coupling the inductors inductively.

19. Apparatus according to claim 17, **characterized in that** the energy content of the frequency transmitted by the control unit to the telemetry unit charges an accumulator or a capacitor for the purpose of providing energy to the implant.

20. Apparatus for controlling the human blood sugar level, comprising:
an implantable apparatus according to one of the claims 16 - 19 for measuring the human blood sugar level, wherein the measured values can be transmitted by a telemetry unit to a control unit located extracorporeally,
an extracorporeal insulin pump for injecting insulin through the peritoneum, and
a regulator integrated into the control unit which controls the insulin pump subject to the measured values in such a way that the desired blood sugar level is attained.

## Revendications

1. Dispositif pour mesurer le taux de sucre sanguin chez l'homme,
avec un cathéter, dont l'extrémité libre peut être placée dans un vaisseau sanguin, le cathéter comprenant au moins un guide de lumière,
avec une source lumineuse, pour coupler la lumière dans le au moins un guide de lumière,
avec un point de mesure à l'extrémité libre du cathéter, auquel la lumière sort du au moins un guide de lumière, la lumière étant dispersée par le sang et/ou guidée par le sang et la lumière, dispersée et/ou guidée dans le volume, est de nouveau couplée au au moins un guide de lumière,
avec un détecteur, pour enregistrer la lumière retournée,
avec une unité de calcul, pour évaluer la lumière enregistrée par le détecteur,
avec un dispositif de nettoyage prévu à l'emplacement de mesure, pour éliminer les parties de tissu s'étant déposées et provenant du sang,
**caractérisé en ce que**,
le dispositif de nettoyage est formé d'un actionneur pouvant être commandé, qui libère ou débarrasse par essuiement une ouverture de sortie de lumière du au moins un guide de lumière lors de la commande.

2. Dispositif selon la revendication 1, **caractérisé en ce que** le dispositif de nettoyage est susceptible d'être commandé par l'unité de calcul.

3. Dispositif selon l'une des revendications 1 à 2, **caractérisé en ce que** le déplacement de l'actionneur s'effectue au moyen d'un alliage à mémoire de forme, au moyen d'un entraînement thermopneumatique, au moyen d'un entraînement électrostatique, ou bien au moyen d'un rotor.

4. Dispositif selon l'une des revendications 1 à 3, **caractérisé en ce que** l'actionneur est formé d'un piston guidé dans une ouverture ajustée précisément, ménagée à l'extrémité libre du cathéter, le guidage se faisant entre une position limite, en affleurement avec la surface du cathéter et une position en retrait par rapport à la surface du cathéter, et **en ce que**, à la position en retrait, une ouverture de sortie de lumière du au moins un guide de lumière est dégagée.

5. Dispositif selon la revendication 4, **caractérisé en ce que** le piston est guidé radialement dans le cathéter.

6. Dispositif selon la revendication 4, **caractérisé en ce que** le piston est guidé axialement dans le cathéter.

7. Dispositif selon l'une des revendications 4 à 6, **caractérisé en ce que** le piston est susceptible d'être entraîné par un micromoteur muni d'un entraînement à vilebrequin, et **en ce que** la ligne de commande est formée d'une ligne de commande électrique.

8. Dispositif selon l'une des revendications 4 à 6, **caractérisé en ce que** le piston est susceptible d'être entraîné au moyen d'un aimant de manoeuvre.

9. Dispositif selon l'une des revendications 1 à 8, **caractérisé en ce que** la commande s'effectue au moyen d'une ligne de commande électrique.

10. Dispositif selon l'une des revendications 1 à 8, **caractérisé en ce que** la ligne ou conduite de commande est formée d'une conduite hydraulique ou pneumatique, reliée fonctionnellement au piston, dans le boîtier susceptible d'être implanté étant intégré un entraînement de manoeuvre, pour produire une pression sur la conduite de commande.

11. Dispositif selon le préambule de la revendication 1, **caractérisé en ce que** le dispositif de nettoyage est un générateur d'ultrasons pouvant être commandé, émettant des ondes ultrasonores lors de la commande, de manière que l'ouverture de sortie de lumière du au moins un guide de lumière soit dégagé des dépôts lorsque commande en est faite.

12. Dispositif selon l'une des revendications 1 à 11, **caractérisé en ce que** l'énergie, nécessaire pour la commande, est fournie à l'emplacement de mesure sous forme électrique, thermique, optique, mécanique, hydraulique ou pneumatique.

13. Dispositif selon l'une des revendications 1 à 12, **caractérisé en ce que** la mesure à l'emplacement de mesure se fait par transmission ou par réflexion diffuse depuis la surface du cathéter.

14. Dispositif selon l'une des revendications 1 à 13, **caractérisé en ce que** le cathéter est enveloppé de matériaux biocompatibles.

15. Dispositif selon la revendication 14, **caractérisé en ce que** le cathéter et le guide de lumière sont réalisés d'une seule pièce à partir d'un matériau massif.

16. Dispositif selon l'une des revendications 1 à 15, **caractérisé en ce que** la source de lumière, le détecteur et l'unité de calcul sont intégrés, conjointement avec un accumulateur d'énergie servant à l'alimentation en tension, dans un implant.

17. Dispositif selon la revendication 16, **caractérisé en ce que**, dans l'implant, est intégrée une unité de télémétrie, à l'aide de laquelle, entre l'unité de calcul et une unité de contrôle se trouvant à l'extérieur du corps, peut être établi une transmission de données et/ou une transmission d'énergie, à l'aide d'une tête de détection prévue à cette fin.

18. Dispositif selon la revendication 17, **caractérisé en ce que** l'unité de télémétrie et la tête de détection présentent un circuit oscillant ayant chacun une inductance, le circuit oscillant pouvant être désaccordé du côté de l'unité de télémétrie et le circuit oscillant pouvant être désaccordé du côté de la tête de détection pour effectuer une transmission de données, et **en ce que** la transmission de données se fait sans contact, par couplage inductif des inductances.

19. Dispositif selon la revendication 17, **caractérisé en ce que** la teneur en énergie de la fréquence transmise de l'unité de contrôle à l'unité de télémétrie charge un accumulateur ou un condensateur devant assurer l'alimentation en énergie de l'implant.

20. Dispositif pour la régulation du taux de sucre sanguin chez l'homme,
avec un dispositif implantable selon l'une des revendications 16 à 19, pour mesurer le taux de sucre sanguin chez l'homme, sachant que les valeurs de mesure peuvent être transmises par une unité de télémétrie à une unité de contrôle se trouvant à l'extérieur du corps,
avec une pompe à insuline extracorporelle, pour injecter de l'insuline à travers le péritoine, et
avec un régulateur intégré dans l'unité de contrôle, qui, en fonction des valeurs de mesure, commande la pompe à insuline, de manière que le taux de sucre sanguin souhaité soit réglé.
